# EUROPEAN PATENT APPLICATION

(11) **EP 1 834 627 A1**
(43) Date of publication of application: **19.09.2007**
(21) Application number: 07003006.9
(22) Date of filing: 13.02.2007
(51) Int. Cl.: A61K 8/34, A61K 8/42, A61K 8/43, A61K 8/49, A61Q 17/00

(54) **Skin conditioning compositions and methods of use thereof**

(30) Priority: 14.02.2006 US 773193 P; 30.12.2006 US 647799
(71) Applicant: JR Chemicals, Milford CT 06460 (US)
(72) Inventor: Ramírez, José E., Trumbull, CT 06611 (US); Faryniarz, Joseph, Middlebury, CT 06762 (US)
(74) Representative: Laufhütte, Dieter

(57) **Abstract**

Compositions and methods of treating skin irritation are disclosed where a conditioning composition is applied to the skin of a user. The compositions and methods are useful to prevent, reduce and/or eliminate irritation of the skin subjected to topical treatment. The conditioning compositions comprise at least one anaesthetic, one analgesic, one cooling constituent and one antimicrobial agent.

## Description

### CROSS REFERENCE TO RELATED APPLICATION

This Application claims priority benefit of U.S. Provisional Application No. 60/773,193 filed February 14, 2006 the entire disclosure of which is incorporated herein by this reference.

### BACKGROUND

### Technical Field

This disclosure relates to the use of conditioning compositions for pharmaceutical and cosmeceutical purposes. The disclosure further relates to certain skin treatment systems, and, more particularly, to the topical application of conditioning compositions to alleviate irritation caused by one or more irritants in a topical composition.

### Background of the Invention

Skin treatments that alleviate and/or cure a multitude of skin conditions routinely apply compositions to skin to thwart undesirable skin conditions. Unfortunately, with topical treatments come a multitude of adverse events affecting both the skin of the user and the treatment itself. For example, users may adversely react to the active ingredients in the topical formulations, which may irritate the skin causing discomfort, pain, or the like. Furthermore, users may adversely react to the various constituents of the topically applied formulations such as the solvents or various excipients which make up the formulation. This may further irritate the skin. Together, these various factors may significantly decrease the effectiveness of the skin treatment resulting in the need to discontinue or change the treatment to obtain a desired beneficial effect, or in some cases continue treatment subjecting the patient to prolonged discomfort.

Accordingly, there remains room for improvement in skin treatment regimens that topically treat skin. What are needed are new skin care compositions and methods for conditioning skin before, after, or both before and after application of one or more topically applied active ingredients or compositions.

### SUMMARY

Dermatological conditions such as skin irritations are prevented and/or treated in accordance with the present disclosure by the topical application of one or more conditioning compositions. Such conditioning compositions can prevent and/or diminish skin irritation caused by the application of one or more active ingredients thereto. Depending on the nature of conditioning compositions, they can be applied before, after, or both before and after application of a topical composition that may have one or more, negative side effects such as skin irritation. In embodiments, the present conditioning compositions include constituents such as anesthetics, analgesics, menthol and/or camphor or derivatives thereof in amounts suitable to prevent and/or diminish skin irritation. The conditioning compositions are suitable for dispensing in formulations suitable for direct topical application to the skin of the user such as toners, lotions, cleansers, gels, or the like.

In addition, dermatological treatment regimens in accordance with the present disclosure can improve characteristics of a user's skin. The regimens include the application of one or more conditioning compositions and one or more corrective composition suitable for treating one or more skin conditions. Suitable corrective compositions include, for example, benzoyl peroxide serums and formulations for treating acne vulgaris.

In a related aspect, the present disclosure is directed towards prophylactic treatment of skin prior to the application of one or more benzoyl peroxide serums.

These and other aspects of this disclosure will be evident upon reference to the following detailed description.

### DETAILED DESCRIPTION OF PREFERRED EMBODIMENTS

The present compositions and methods provide formulations for alleviating irritation caused by the topical skin treatment with one or more topical compositions. Such methods involve sequentially applying to an area of the skin, an effective amount of one or more conditioning compositions, and/or one or more corrective compositions. These compositions are topically applied to the skin in sequential order starting with the conditioning composition, and then followed by the corrective compositions.

As used herein the word "treat," "treating" or "treatment" refers to using the compositions of the present disclosure either prophylactically to prevent outbreaks of undesirable dermatological symptoms such as irritation, or therapeutically to ameliorate an existing undesirable dermatological condition. Treatment regimens in accordance with the present disclosure improve skin characteristics through sequential application of preselected skin care compositions to the skin of a user. As used herein the word "conditioning composition" refers to using the compositions of the present disclosure to prophylactically prevent outbreaks of undesirable dermatological symptoms such as irritation, or to ameliorate an existing undesirable dermatological condition caused by an irritant or the like. When conditioning compositions are used in conjunction with a corrective composition, they may produce a beneficial effect. As used herein the word "corrective composition" refers to using the compositions, which have an active ingredient for treating any undesirable dermatological condition. Application of the conditioning composition in combination with the one or more corrective compositions provides improved effectiveness of the corrective composition compared to application of the corrective composition alone. This is especially true for embodiments where both the conditioning composition and corrective compositions contain antimicrobial agents. For example, a positive benefit is achieved by contacting skin with a combination of antimicrobials such as chlorhexidine diacetate in the conditioning compositions and benzoyl peroxide in a corrective serum composition.

In embodiments, the treatment includes the application of an effective amount of one or more conditioning compositions (e.g., gel, toner and/or lotion) followed by application of an effective amount of one or more corrective compositions (e.g., benzoyl peroxide serum). The treatment may be followed by additional application of an effective amount of one or more additional conditioning compositions (e.g., gel, toner and/or lotion). Such treatments may prevent, alleviate, or eliminate pain caused by the application of the corrective composition.

### Conditioning Compositions

Throughout the treatment regimen of the present disclosure, skin improvement may be slowed or worsened by discomfort to skin due to the application of a topical composition. Conditioning compositions can be applied in amounts effective to make skin more receptive to the corrective step. Such compositions may sooth skin, eliminate and/or alleviate the feeling of pain or discomfort in a user. Thus, the treatment regiment of the present disclosure includes the step of preparing skin to make it more receptive to the corrective step by applying conditioning compositions.

In embodiments, the conditioning composition may include one or more topical anesthetics to prevent pain or irritation caused by the application of one or more irritants to skin such as a corrective composition or constituent thereof. Topical anesthetics, all of which are believed to be useful in accordance with the present disclosure, can be present in any amount sufficient to effectively reduce pain upon tissue penetration. Non-limiting examples of suitable anesthetic constituents include: ambucaine, amolanone, amylocaine, benoxinate, betoxycaine, biphenamine, bupivacaine, levo-bupivacaine, butacaine, butamben, butanilicicaine, butethamine, butoxycaine, carticaine, cocaethylene, cyclomethycaine, dibucaine, dimethisoquin, dimethocaine, diperodon, dyclonine, ecgonidine, ecgonine, ethyl aminobenzoate, ethyl chloride, levo-etidocaine, etidocaine, dextro-etidocaine, .beta.-eucaine, euprocin, fenalcomine, fomocaine, hexylcaine, hydroxyprocaine, hydroxytetracaine, isobutyl p-aminobenzoate, leucinocaine mesylate, levoxadrol, meperidine, levo-mepivacaine, mepivacaine, meprylcaine, metabutoxycaine, methyl chloride, myrtecaine, naepaine, octacaine, orthocaine, oxethazaine, parethoxycaine, phenacaine, phenol, a pipecoloxylidide, piperocaine, piridocaine, polidocanol, pramoxine, sameridine, prilocaine, propanocaine, proparacaine, propipocaine, propoxycaine, pseudococaine, pyrrocaine, quinine urea, risocaine, ropivacaine, levo-ropivacaine, salicyl alcohol, tetracaine, tolycaine, trimecaine, veratridine, zolamine, a 2-alkyl-2-alkylamino-2',6'-acetoxylidide compound, a glycerol 1,2-bis-aminoalkyl ether compound, a benzisoxazole compound, an O-aminoalkylsalicylate compound, a heterocyclic phenoxyamine compound, a 2-substituted imidazo(1,2-A)pyridine compound, a 3-aryl substituted imidazo(1,2-A)pyridine compound, a polyorganophosphazene compound, a tertiary-alkylamino-lower acyl-xylidide compound, an amidinourea compound, a 3-(5'-adenylate) of a lincomycin compound, a 3-(5'-adenylate) of a clindamycin compound, an N-substituted derivative of a 1-(4'-alkylsulfonylphenyl)-2-amino-1,3-propanediol compound, a tertiary aminoalkoxyphenyl ether compound, an adenosine compound, adenosine, adenosine monophosphate, adenosine diphosphate, or adenosine triphosphate, a lauryl polyglycol ether compound, a 2-(.omega.-alkylaminoalkyl)-3-(4-substituted-benzylidene) phthalimidine compound, a 2-(.omega.-dialkylaminoalkyl)-3-(4-substituted-benzylidene)phthalimidine compound, an N,N,N-triethyl-N-alkyl ammonium salt, an L-N-n-propylpipecolic acid-2,6-xylidide compound, a polymer comprising repeating units of one or more local anesthetic moieties, an N-substituted 4-piperidinecarboxamide compound, an N-substituted 4-phenyl-4-piperidinecarboxamide compound; and a pharmaceutically acceptable derivative thereof, and combinations of these anesthetic agents. In embodiments, pharmaceutically acceptable salts of these anesthetic constituents are suitable for use in accordance with the present disclosure. In embodiments, salts include the amine addition salts of inorganic and organic acids, e.g., the hydrochloride, hydrobromide, sulfate, oxalate, fumarate, citrate, malate, propionate and phosphate salts. In embodiments, suitable anesthetics can be combined with menthol. In embodiments, benzyl alcohol is a suitable anesthetic for use in accordance with the present disclosure. In embodiments, benzyl alcohol, lidocaine, pramoxine HCL, and combinations thereof may be included in amounts sufficient to eliminate the feeling of pain or irritation in a patient. In embodiments, the anesthetic may be used in an amount of about 0.01% to about 5.0%, based upon the total weight of the conditioning composition, in embodiments from about 0.25% to about 3% by weight of the conditioning composition.

In embodiments, the conditioning composition may optionally include an analgesic or pain killer in amounts sufficient to reduce or eliminate pain or irritation caused by the application of one or more corrective compositions or constituent thereof to skin. Suitable analgesics include any drug used to relieve pain. Such drugs may act in a variety of ways on the peripheral and/or central nervous system to achieve analgesia. Non-limiting examples of suitable analgesics include acetaminophen, the nonsteroidal anti-inflammatory drugs (NSAIDs) such as the salicylates, narcotic drugs such as morphine and derivatives thereof, synthetic drugs with narcotic properties, and various others including drugs not normally considered analgesics used to treat neuropathic pain syndromes such as tricyclic antidepressants and anticonvulsants like gabapentin. Other analgesic compounds suitable for use in accordance with the present disclosure are opioid analgesics. Such opioid analgesics are well known to those skilled and in the art and include, for example codeine, morphine, dihydrocodeine, butorphanol, etc. Thus, the compositions of the present disclosure include those where the analgesic compound is an opioid analgesic, a non-opioid analgesic, or an NSAID, including physiologically active and pharmaceutically acceptable salts and isomers thereof. The analgesic may be used in an amount of about 0.01% to about 5.0%, based upon the total weight of the conditioning composition.

Another component that may be present in the conditioning composition in accordance with the present disclosure is a cooling agent in amounts sufficient to provide a fresh, cooling feeling to the user. Suitable non-limiting examples of coolants include: menthol; eucalyptus oil; peppermint oil; cyclohexanol, 5-methyl-2-(1-methylethenyl)-, available from Takasago International Corporation, Tokyo under the tradename, COOLACT; 6-Isopropyl-9-methyl-1,4-dioxaspiro-(4,5)decane-2-methanol, (I)-menthone glycerol ketal (Menthone Glycerin Acetal) available from Haarmann & Reimer ("H&R") under the tradename, FRESCOLAT MGA; 5-methyl-2-(1-methyl ethyl)-cyclohexyl-2-hydroxypropionate, I-menthyl lactate, acid/-menthyl ester (Menthyl Lactate) available from H&R under the tradename, FRESCOLAT ML; FRESCOLTA PLUS also available from H&R; menthyl pyrrolidone carboxylate (Menthyl PCA) available from Quest International UK Limited under the tradename, QUESTICE, and mixtures thereof. The cooling agent may be used in an amount of about 0.01 % to about 0.5%, based upon the total weight of the conditioning composition. In embodiments, menthol may be used as a coolant in amounts sufficient to act as a local anesthetic and/or counterirritant.

Another component that may be present in the conditioning composition in accordance with the present disclosure is a camphor or derivative thereof. Camphor or derivatives thereof may be presents in amounts suitable for providing a soothing effect on the skin of the user. Suitable non-limiting examples of camphor derivatives include: 4-methyl-benzylidene camphor [3-(4'-methyl)benzylidene-bornan-2-one], 3-benzylidene camphor (3-benzylidene-bornan-2-one), polyacrylamidomethylbenzylidene camphor {N-[2(and 4)-2-oxyborn-3-ylidene-methyl)benzyl]acrylamide polymer}, trimonium-benzylidene camphor sulfate [3-(4'-trimethylammonium)-benzylidene-bornan-2-one methyl sulfate], terephthalydene dicamphorsulfonic acid {3,3'-(1,4-phenylenedimethine)-bis(7,7-dimethyl-2-oxo-bicyclo[2.2.1]heptane-1-methanesulfonic acid} or salts thereof, and benzylidene camphorsulfonic acid [3-(4'-sulfo)benzylidene-bornan-2-one] or salts thereof. The camphor constituent may be used in an amount of about 0.01 % to about 0.5%, based upon the total weight of the conditioning composition.

Another component of the conditioning compositions may be an antimicrobial agent in amounts sufficient to minimize or kill microbes on the skin. Any topically applied antimicrobial agent can be used as a constituent of the conditioning composition. Suitable non-limiting examples of antimicrobial agents for use in accordance with the present disclosure include: benzethonium chloride, chlorhexidine diacetate, (-p-Chloro-3, 5-m-xylenol) ("PCMX")(also known as "chloroxylenol"), and combinations thereof. The antimicrobial constituent may be used in an amount of about 0.01% to about 0.5%, based upon the total weight of the conditioning composition.

Solvents useful for preparing the present conditioning compositions include any solvent capable of solubilizing the constituents described herein. Such solvents include short chain alkyl esters, ethers, aldehydes, ketones or alcohols of benzoic acid, benzyl alcohol, salicylic acid, phenol or phathalic acid. Other suitable solvents include aryl esters, ethers, aldehydes, ketones and alcohols of benzoic acid, benzyl alcohol, salicylic acid, phenol and phthalic acid. In embodiments, the conditioning compositions include one or more of the following solvents: ethoxyethanol and/or SDA alcohol. In embodiments, ethoxyethanol may be supplied in an amount of about 10% of the formulation. In embodiments, SDA alcohol may be supplied in an amount of about 20-40% of the formulation. In embodiments, water may be used as a solvent, either alone, or in combination with other solvents. In embodiments, the solvent may be supplied in an amount of about 5.0% to about 90%, based upon the total weight of the conditioning composition.

In embodiments, conditioning products containing anesthetics, analgesics, antimicrobial, cooling and/or solvent constituents in accordance with the present disclosure can be in the form of solutions, emulsions (including microemulsions), suspensions, creams, lotions, gels, powders, or other typical solid or liquid compositions used for treatment of skin irritation. Such compositions may contain, in addition to anesthetic, analgesic, antimicrobial, cooling, and solvent constituents in accordance with this disclosure, other ingredients typically used in such products, such as moisturizers and hydration agents, penetration agents, preservatives, emulsifiers, natural or synthetic oils, surfactants, detergents, gelling agents, emollients, antioxidants, fragrances, fillers, thickeners, waxes, odor absorbers, dyestuffs, coloring agents, powders, viscosity-controlling agents and water, and optionally including anti-itch actives, botanical extracts, conditioning agents, darkening or lightening agents, glitter, humectants, mica, minerals, polyphenols, silicones or derivatives thereof, sunblocks, vitamins, and phytomedicinals.

As an illustrative example of a conditioning composition, toner products can be formulated with the following constituents:

### Example 1

| **CONSTITUENT** | **% w/w** | **Range % w/w** |
|---|---|---|
| COOLACT 10 | 1 % | 0.05-5% |
| FESCOLAT | 0.25% | 0.05-5% |
| FRESCOLAT PLUS | 0.25% | 0.05-5% |
| Chlorhexadine diacetate | 0.25% | 0-5% |
| Benzyl alcohol | 2% | 0-5% |
| Lidocaine | 2% | 0-5% |
| Pramoxime HCL | 1% | 0-5% |
| Ethoxyethanol | 10% | 5-20% |
| SDA Alcohol | 20-40% | 10-50% |
| Water | Add up to 100% | 25-99% |

### CORRECTIVE COMPOSITIONS

Throughout the treatment regimen of the present disclosure, skin improvement may be obtained by application of one or more corrective compositions. Corrective compositions can be applied in amounts effective to treat, alleviate and/or cure an undesirable skin condition. For example, such corrective compositions may cure acne Vulgaris by the topical application of one or more benzoyl peroxide serums. Thus, the treatment regiment of the present disclosure includes the step of treating skin to provide a beneficial effect. Suitable corrective compositions include serums, toners, gels, lotions and combinations thereof, which may be applied to the skin after the application of the conditioning composition.

The corrective compositions may contain active ingredients in an effective amount to prevent or improve one or more skin conditions. As used herein with respect to active ingredients in corrective compositions, effective amount refers to an amount of active ingredient that is sufficient to induce a particular positive benefit to skin. The positive benefit can be health-related, or it may be more cosmetic in nature, or it may be a combination of the two. The particular active ingredient or ingredients employed, and the concentration in the compositions, generally depends on the purpose for which the composition is to be applied. For example, organic peroxides such as benzoyl peroxide can be selected to treat acne Vulgaris. In embodiments, the positive benefit is achieved by contacting skin with a combination of antimicrobials such as chlorhexidine diacetate and benzoyl peroxide. However, any active ingredient suitable for treating any skin condition can be used in treatment regimens in accordance with the present disclosure.

In embodiments, suitable corrective compositions for use as active drug in accordance with the present disclosure provide a solvent vehicle formulation for the treatment of acne in which the major active ingredient is benzoyl peroxide. The active ingredients are provided in clear product forms. Such clear product forms include serums, toners, pump or aerosol sprays, clear gels, sticks, creams, lotions and mousses. The clear product forms improve the effectiveness of the ingredients applied, allowing the use of lower levels and providing quicker patient response. Also the use of these vehicle solvents in emulsion product forms allows levels of benzoyl peroxide in oil/water emulsions to exceed the solubility parameters of benzoyl peroxide in oil/water systems without the solvent vehicle, resulting in a slurry type application which will also benefit with increased antimicrobial activity due to the solubilized fraction of the benzoyl peroxide.

In embodiments, suitable corrective compositions for use in accordance with the present disclosure are described in U.S. Application No. 60/660,386 filed on March 10, 2005 entitled *Benzoyl Peroxide Serums;* U.S. Application No. 60/660,387 filed on March 10, 2005 entitled *Stable Organic Peroxide Compositions;* and U.S. Application No. 60/695,223 filed on June 29, 2005 entitled *Stable Organic Peroxide Compositions.* All of these applications are herein incorporated by reference in their entirety.

In embodiments, corrective compositions in accordance with the present disclosure can be in the form of solutions, emulsions (including microemulsions), suspensions, creams, lotions, gels, powders, or other typical solid or liquid compositions used for treatment of skin irritation. Such compositions may contain antimicrobial, cooling, solvent constituents and, other ingredients typically used in such products, such as moisturizers and hydration agents, penetration agents, preservatives, emulsifiers, natural or synthetic oils, surfactants, detergents, gelling agents, emollients, antioxidants, fragrances, fillers, thickeners, waxes, odor absorbers, dyestuffs, coloring agents, powders, viscosity-controlling agents and water, and optionally including anti-itch actives, botanical extracts, conditioning agents, darkening or lightening agents, glitter, humectants, mica, minerals, polyphenols, silicones or derivatives thereof, sunblocks, vitamins, and phytomedicinals.

Non-limiting examples of corrective compositions which may be used in combination with the conditioning compositions in accordance with this disclosure are listed below.

### EXAMPLES 2 - 12

### Clear serums were formulated having the following compositions:

| Example # | 2 | 3 | 4 | 5 | 6 | 7 | 8 | 9 | 10 | 11 | 12 |
|---|---|---|---|---|---|---|---|---|---|---|---|
| Benzyl Peroxide 98% | 10 | 10 | 10 | 10 | 8 | 8 | 8 | 8 | 8 | 8 | 8 |
| Dimethyl Isosorbide | | | 30 | 40 | 46 | 43.5 | 45 | 45 | 45 | 45 | 45 |
| Benzyl Benzoate | 85 | | 60 | | | | | | 18.5 | | |
| Acetone | 5 | 5 | | | | | | | | | |
| Extend 226 | | 85 | | 30 | 46 | 43.5 | 23.5 | 18.5 | -- | 37 | 34 |
| Benzyl alcohol | | | | 20 | | | 23.5 | 18.5 | 18.5 | | 3 |
| Ethoxy ethanol | | | | | | 5 | | 10 | 10 | 10 | 10 |

### EXAMPLES 13

### An emulsion was formulated having the following composition:

| Ingredient | Amount |
|---|---|
| Water | 67.6% |
| Steareth 20 | 0.8% |
| Glycerine | 4.0% |
| Benzoyl Peroxide 74% | 7.0% |
| Benzyl benzoate | 10.0% |
| Cyclomethicone | 5.0% |
| Ethylene diamine tetraacetic acid disodium salt (EDTA) | 0.1% |
| Stearyl alcohol | 4.0% |
| Steareth 2 | 1.5% |

### EXAMPLE 14

### Another suitable corrective composition contains:

| Ingredient | Amount |
|---|---|
| Benzoyl Peroxide | 6.25% |
| Benzoyl benzoate | 42.45% |
| Dimethyl isosorbide | 40.00% |
| Vitamin E Acetate | 0.5% |
| BHT | 0.8% |
| Ethoxy diglycol | 10.0% |

The following non-limiting examples further illustrate methods in accordance with this disclosure.

### Example 15

A 15 year old male is suffering from acne. The treatment is problematic because a corrective benzoyl peroxide serum is irritating the treated skin causing pain. A treatment regimen is started where toner of Example 1 is applied prior to application of the benzoyl peroxide serum. The patient does not experience any additional discomfort from using the corrective composition and treatment is continued.

### Example 16

A 16 year old female is suffering from acne. The treatment is problematic because a corrective benzoyl peroxide serum of Example 8 is irritating the skin and causing pain. A treatment regimen is started where toner of Example 1 is applied before and after application of the corrective composition. The patient does not experience any additional discomfort from using the corrective composition and treatment is continued.

While several embodiments of the disclosure have been described, it is not intended that the disclosure be limited thereto, as it is intended that the disclosure be as broad in scope as the art will allow and that the specification be read likewise. Therefore, the above description should not be construed as limiting, but merely as exemplifications of embodiments. Those skilled in the art will envision other modifications within the scope and spirit of the claims appended hereto.

## Claims

1. A topical skin composition for inhibiting skin irritation comprising:
one or more anesthetic constituents in an effective amount to inhibit the feeling of skin irritation;
one or more analgesic constituents in an effective amount to inhibit the feeling of skin irritation;
one or more cooling constituents in an effective amount to provide a cooling sensation to skin; and
one or more antimicrobial constituents in an effective amount to inhibit microbes on the skin.

2. A composition as in claim 1 wherein the one or more analgesic constituents are selected from the group consisting of acetaminophen, nonsteroidal anti-inflammatory drugs (NSAIDs), narcotic drugs, opioid analgesics, drugs used to treat neuropathic pain syndromes and mixtures thereof.

3. A composition as in claim 1 wherein the one or more anesthetic constituents is selected from the group consisting of ambucaine, amolanone, amylocaine, benoxinate, betoxycaine, biphenamine, bupivacaine, levo-bupivacaine, butacaine, butamben, butanilicicaine, butethamine, butoxycaine, carticaine, cocaethylene, cyclomethycaine, dibucaine, dimethisoquin, dimethocaine, diperodon, dyclonine, ecgonidine, ecgonine, ethyl aminobenzoate, ethyl chloride, levo-etidocaine, etidocaine, dextro-etidocaine, β-eucaine, euprocin, fenalcomine, fomocaine, hexylcaine, hydroxyprocaine; hydroxytetracaine, isobutyl p-aminobenzoate, leucinocaine mesylate, levoxadrol, meperidine, levo-mepivacaine, mepivacaine, meprylcaine, metabutoxycaine, methyl chloride, myrtecaine, naepaine, octacaine, orthocaine, oxethazaine, parethoxycaine, phenacaine, phenol, a pipecoloxylidide, piperocaine, piridocaine, polidocanol, pramoxine, sameridine, prilocaine, propanocaine, proparacaine, propipocaine, propoxycaine, pseudococaine, pyrrocaine, quinine urea, risocaine, ropivacaine, levo-ropivacaine, salicyl alcohol, tetracaine, tolycaine, trimecaine, veratridine, zolamine, a 2-alkyl-2-alkylamino-2',6'-acetoxylidide compounds, a glycerol 1,2-bis-aminoalkyl ether compounds, benzisoxazole compounds, O-aminoalkylsalicylate compounds, heterocyclic phenoxyamine compounds, 2-substituted imidazo(1,2-A)pyridine compounds, 3-aryl substituted imidazo(1,2-A)pyridine compounds, polyorganophosphazene compounds, tertiary-alkylamino-lower acyl-xylidide compounds, amidinourea compounds, 3-(5'-adenylate) of a lincomycin compounds, 3-(5'-adenylate) of a clindamycin compounds, N-substituted derivatives of 1-(4'-alkylsulfonylphenyl)-2-amino-1,3-propanediol compounds, tertiary aminoalkoxyphenyl ether compounds, adenosine, adenosine monophosphate, adenosine diphosphate, adenosine triphosphate, lauryl polyglycol ether compounds, 2-(ω-alkylaminoalkyl)-3-(4-substituted-benzylidene) phthalimidine compounds, 2-(ω-dialkylaminoalkyl)-3-(4-substituted-benzylidene)phthalimidine compounds, ,N,N-triethyl-N-alkyl ammonium salts, L-N-n-propylpipecolic acid-2,6-xylidide compounds, polymers comprising repeating units of one or more local anesthetic moieties, N-substituted 4-piperidinecarboxamide compounds, N-substituted 4-phenyl-4-piperidinecarboxamide compounds and combinations thereof.

4. A composition as in claim 1 wherein the one or more cooling constituents are selected from the group consisting of menthol, eucalyptus oil, peppermint oil, cyclohexanol, 5-methyl-2-(1-methylethenyl)-, 6-Isopropyl-9-methyl-1,4-dioxaspiro-(4,5)decane-2-methanol, (I)-menthone glycerol ketal (Menthone Glycerin Acetal), 5-methyl-2-(1-methyl ethyl)-cyclohexyl-2-hydroxypropionate, I-menthyl lactate, acid/-menthyl ester (Menthyl Lactate), menthyl pyrrolidone carboxylate (Menthyl PCA) and mixtures thereof.

5. A composition as in claim 1 wherein the one or more antimicrobial constituents are selected from the group consisting of benzethonium chloride, chlorhexidine diacetate, p-Chloro-3, 5-m-xylenol and combinations thereof.

6. A composition as in claim 1 further comprising a solvent.

7. A composition as in claim 1 wherein the solvent is selected from the group consisting of short chain alkyl esters, ethers, aldehydes, ketones, alcohols of benzoic acid, benzyl alcohol, salicylic acid, phenol, phathalic acid and combinations thereof.

8. A composition as in claim 1 wherein the one or more analgesic constituents are present in an amount of about 0.01 % to about 5.0% based upon the total weight of the composition.

9. A composition as in claim 1 wherein the one or more anesthetic constituents are present in an amount of about 0.01 % to about 5.0% based upon the total weight of the composition.

10. A composition as in claim 1 wherein the one or more cooling agents are present in an amount of about 0.01 % to about 0.5% based upon the total weight of the composition.

11. A composition as in claim 1 wherein the one or more antimicrobial agents are present in an amount of about 0.01% to about 0.5% based upon the total weight of the composition.

12. A topical skin composition for inhibiting skin irritation comprising a coolant, Chlorhexadine diacetate, Lidocaine, Pramoxime HCL, and a solvent.

13. A kit comprising:
a conditioning composition comprising: one or more anesthetic constituents in an effective amount to inhibit the feeling of skin irritation;
one or more analgesic constituents in an effective amount to inhibit the feeling of skin irritation;
one or more cooling constituents in an effective amount to provide a cooling sensation to skin; and
one or more antimicrobial constituents in an effective amount to inhibit microbes on the skin; and
a corrective composition.

14. A method of treating skin and reducing skin irritation comprising:
contacting skin in need thereof with an effective amount of conditioning composition to prevent skin irritation; and
contacting skin in need thereof with a corrective composition, wherein skin is less irritated than if contacted by corrective composition alone.

15. A method as in claim 14 wherein the conditioning composition comprises
one or more anesthetic constituents in an effective amount to inhibit the feeling of skin irritation;
one or more analgesic constituents in an effective amount to inhibit the feeling of skin irritation;
one or more cooling constituents in an effective amount to provide a cooling sensation to skin; and
one or more antimicrobial constituents in an effective amount to inhibit microbes on the skin.
